Europäisches Patentamt

European Patent Office

Office européen des brevets

(11) Publication number: **0 223 572**
**A1**

# EUROPEAN PATENT APPLICATION

(21) Application number: 86308887.8

(22) Date of filing: 14.11.86

(51) Int. Cl.⁴: **A 61 K 7/13**

(30) Priority: 15.11.85 US 798772

(43) Date of publication of application: 27.05.87
Bulletin 87/22

(84) Designated Contracting States: **AT BE CH DE ES FR GB
GR IT LI LU NL SE**

(71) Applicant: **Bristol-Myers Company, 345 Park Avenue,
New York New York 10154 (US)**

(72) Inventor: **Massoni, Jack T., 55, S. Carridge Path, Milford
Connecticut 06460 (US)**

(74) Representative: **Jones, Alan John et al, CARPMAELS &
RANSFORD 43 Bloomsbury Square, London, WC1A 2RA
(GB)**

(54) Oxidation hair dye composition with color indication.

(57) The invention provides an oxidation dye composition for
dyeing hair which is temporarily coloured to identify the colour
or colour category produced by the composition after develop-
ment in the presence of an oxidizing agent. Such a composition
indicates to the hair colourist by quick visual inspection the
colour or colour category to which the composition belongs.
The composition comprises oxidation dye intermediate(s)
which react in the presence of an oxidizing agent to develop
colour on hair, and a pigment component which is water-insoluble or substantially water-insoluble and which will be readily
washed out of hair to which it is applied.

# OXIDATION HAIR DYE COMPOSITION WITH COLOR INDICATION

This invention relates to a composition for imparting temporary color to oxidation hair dye compositions. More particularly, this invention, in its most preferred embodiment, relates to system for color coding oxidation hair coloring products which will identify the color or color category to which the products belong and to the corresponding color coded products. The system and products of the aforesaid character signal for the hair colorist by quick visual inspection of the product disposed in a container the color of color category to which the product belongs.

In oxidation hair dye products it is customary to package in one container the dye intermediates necessary for the reaction to produce the color on hair and in a second container the oxidizing agent. These will be mixed together just before use and the mixture is applied to the hair. The mixture of dye intermediates is essentially colorless, or has a weak incidental color that is generally unattractive. The eventual color of the hair dye is developed only after the intermediates are mixed with the oxidizing agent. It is desirable to impart visually attractive color to hair dye intermediates, and then have this color washed out from the hair so that it does not interfere with the appearance and color of the hair that is colored by the dye. Since solutions of hair dye intermediates are colorless or have a weak, incidental color, there is no way of signaling the color or color category to which the dye belongs by quick beforehand visual examination. Therefore, it is also desirable, in addition to imparting a temporary color to hair dye
intermediates, to make that temporary color the same,
or that of the color category, as the dye which will
have been developed by the oxidation hair dye
intermediates after their having been contacted with
the oxidizer.

It has now been found that the above described
objectives can be achieved by incorporating in the
mixture of an essentially colorless or weakly
incidentally colored oxidation dye intermediate a
pigment which will serve to color the mixture and,
preferably, identify the color or category of colors
which can be formed by the dye.

The pigments that can be used in practicing this
invention are generally water insoluble or substantially water insoluble ones.  They are selected so that
they are not deposited on the hair and, therefore,
essentially no coloration of the hair occurs due to
their presence.  After application to the hair the
pigment can be removed by the shampooing which
customarily follows hair dyeing anyway.

The color of the pigment selected for coding purposes can be arbitrary or can be related to the color
that the oxidation dye product will impart to the
hair.  As indicated above the color coding may apply
to a single color or a category of colors.  That is to
say that the color of the pigment employed in any
product may be used to signal the operator that the
product will impart a specific color to the hair or
that it will impart to the hair any one of a family of
related colors.

In the preferred form of this invention the color pigment selected will at least in some degree reflect the color that product will give to the hair. Moreover for practical reasons the pigment selected can be most suitably used as means of identifying a category of colors rather than a single color. The latter feature will preferably be used in conjunction with a pigment whose color is somewhat related to the color which the products in a particular color category will impart to the hair. For a convenience of description, a system is described below, that employs five color categories that are readily recognizable by a skilled hair colorist. It is to be understood, however, that this invention will not be limited to such grouping of colors.

In a preferred form of this invention pigments are employed to identify the following color categories: Blue-Gray, Violet, Red-Violet, Red-Orange and Gold. In this case also the color of the pigments is in fact related to the literal color designation of the color category. Thus, for example, the Blue-Gray color category will be identified by a pigment which is in fact colored do some shade of Blue-Gray.

Within each color category there are included in the illustrative system a family of intermediate oxidation dye products that are related to each other in that they are all identified by the same color code designation e.g., Blue-Gray, Violet, etc. The individual members of these families, however, differ from each other in the shade (i.e. the degree of lightness or darkness) of the color representing the color category to which it belongs. Of course, each individual product could be identified by a differently colored pigment. This, however, would unduly multiply the

- 4 -                    0223572

number of different colors that have to be employed.
As a practical matter it has been convenient to employ
only 5 different colored pigments to in combination
with a luminosity rating.  The color category to which
a product belongs is identifiable by the visual appearance of the product.  It's particular luminosity
is identifiable by a number that can be applied to the
container for the product.

This invention is particularly useful in busy hair
salons.  If the hairdresser were to pick up a product
of the wrong color by mistake, the color of the product would immediately warn him of the error.  For most
purposes it would be sufficient in this regard for the
skilled hair colorist that he would be warned that the
product does not fall in the color category that he
means to use.

Any variety or types of pigments can be employed
in the practice of the present invention.  Most pigments in use today fall into five groups that can be
described as follows:

1.  Organic Pigments - Concentrated organic coloring
    matter containing no salt forming groups.

2.  Toners - Concentrated coloring matters produced by
    reaction of a water soluble dye with an appropriate
    precipitant.

3.  Lakes - Similar to Toners but precipitated in the
    presence of a substrate such as alumina, the substrate being a necessary and integral part of the
    product.

4.  Extended Pigments - Pigments or Toners diluted
    with an extender which is not an integral part of
    the pigment.

5.  <u>Inorganic Pigments</u> - Colored, insoluble compounds
    of mainly inorganic composition.

Those pigments that have been found particularly
suitable in the practice of the present invention are
the inorganic pigments and the organic pigments.  Of
special interest for esthetic reasons are the pearlized
pigments.  These pigments contain mica platelets which
have precipitated onto them thin layers of a pigment,
especially inorganic pigments e.g., titanium dioxide,
iron oxides etc.  When incorporated in the intermediate
oxidation dye products of this invention they give them
an esthetically pleasing pearlized appearance.

In accordance with this invention the single pigments or a combination of pigments are utilizable depending on the color and/or effect desired.  For
example, when a pearlized pigment of a suitable color
is not available it is often advantageous to add to
this pigment the pearlized titanium dioxide pigment to
give the former a pearlized appearance.  In the following Table I examples of pigments or combination of pigments are given by way of illustration, that may be
used to practice this invention.

## TABLE I

| CHEMICAL | COLOR PRODUCED |
|---|---|
| $TiO_2$ coated mica with iron oxide | Violet Red Pearl |
| $TiO_2$ coated Mica with Carmine | Reddish-Blue Pearl |
| Ultramarine Violet | |
| $Na_5H\ Al4\ Si_6S_4O_{24} \cdot H_2O$ | Violet |
| Pigment Blue 15 | |
| C.I. Constitution No. 74160 | Blue |

C.I. Pigment Blue 15 (Bright blue)

Pigment Violet 23
   C.I. Constitution No. 51319          Violet

C.I. Pigment Violet 23 (bluish violet)

Pigment Orange 16
   C.I. Constitution No. 21160          Orange

C.I. Pigment Orange 16 (bright orange - reddish orange)

In preparing the products of the present invention, as previously indicated, the pigment component will be incorporated in an intermediate oxidation dye composition. As used herein the term "pigment component" refers to a single pigment or a combination of pigments, as the case may be. The quantity of pigment component which is contained in the intermediate oxidation dye composition of this invention can vary with the pigment or pigments selected and the color desired. Generally, however, the pigment component constitutes from about 0.01% to about 10.0% by weight based on the total weight of the intermediate oxidation dye composition with the preferred range being from about 0.01% to about 1.0% on the same weight basis.

The intermediate oxidation dye compositions to which the pigment component is added in accordance with this invention, can be any of the conventional intermediate oxidation dye compositions that are well known to those skilled in the hair dye art. These can contain one or more meta components in combination with one or more para components which are intended to couple with each other when mixed with an oxidizing agent to generate a color on the head. In addition, it may also contain one or more oxidation dye intermediates that are self coupleable in the presence of oxidation dye or one or more direct dyes that are used to modify the principal color developed by the reaction of the oxidation dyes.

The intermediate oxidation dye composition employed herein may also contain agents which are designed to improve the stability of the composition, adjust the pH of the composition, to improve the organoleptic properties of the product, to facilitate the application of the product to the hair or to improve the dyeing characteristics.  Other components of the intermediate oxidation dye composition used for the present purposes will be determined by the form of the product e.g., solution, cream, gel, etc.

In a preferred aspect of this invention the intermediate oxidation dye composition with the pigment component added will have the form of a cream based product or a gel.  Cream based and gel hair dyes are well known in the hair dyeing art.  The reason that they are preferred is that pigments will remain suspended, i.e. well distributed throughout the viscous cream or gel base, whereas in watery fluid bases the pigments will settle out and will have to be shaken up before use.  The gel can be prepared by adding a molten mixture of the following:  oleic acid, Lauramide DEA or Cocamide DEA, cetyl alcohol, Steareth-21 or Polysorbate-20; to an aqueous mixture of dye intermediates and antioxidants.  While stirring, ammonium hydroxide is added to this mixture.  The oleic acid is then neutralized by the ammonium hydroxide forming the gel.  The other above mentioned components also contribute to the gel formation.

The pigments can be incorporated into the formulation by either dispersing them before or after the ammonium hydroxide is added.

The following examples are given to further illustrate the present invention.  It is to be understood, however, that the invention is not limited thereto.  For example, many other colorants may be used than those specified in the Examples to achieve similar results.

In the procedure for preparing formulation of Examples #1-5, dye intermediates and antioxidants, such as sodium sulfite, EDTA, m-aminophenol, 1-naphthol, N,N-bis(2-hydroxyethyl) -p-phenylene diamine sulfate, resorcinol, p-phenylenediamine, p-aminophenol, 4-amino-2-hydroxytoluene, and 2-methyl resorcinol are added to the batch vessel and dissolved in 50% of the total deionized water at 60-80°C with continuous stirring using a laboratory mixer and propeller. Premix "A" is prepared by heating any of the following ingredients: oleic acid, Cocamide DEA or Lauramide DEA, cetyl alcohol, Steareth-21, Polysorbate 20, stearyl alcohol, stearic acid, lanolin alcohol, sodium lauryl sulfate, polyoxyethylene 40 stearate, behenic acid, behenyl alcohol, lauryl amine oxide, polyoxyethylene 50 lanolin derivative, beeswax, mineral oil, oleyl alcohol, or synthetic wax to 70°C until all solids have melted. The above names are based on the CTFA Cosmetic Ingredient Dictionary. Premix "A" is added to the batch tank with continuous stirring. The remaining deionized water (cold) is added to the batch tank with continuous stirring. The pigment components, such as Ultramarine Blue; mica and titanium dioxide, Ultramarine Violet, titanium dioxide Coated Mica with iron oxide, mica and titanium dioxide and Carmine, mica and titanium dioxide and $Fe_2O_3$, and Pigment Orange 16 and fragrance are added to the batch tank with continuous stirring. The ammonium hydroxide or mono-ethanol amine is added to the batch with continuous stirring. The batch will thicken, then is stirred for an additional 10-15 minutes or until batch is uniform. Alternately the pigment components can be added immediately after the addition of ammonium hydroxide, or mono-ethanol amine.

## Example 1 - Light Smokey Brown
### (Blue-Gray class shade)

|  | WT % |
|---|---|
| mono-ethanol amine | 11.000 |
| Stearyl Alcohol | 13.000 |
| Stearic Acid | 3.000 |
| Lanolin Alcohol | 1.000 |
| Sodium Lauryl Sulfate | 1.000 |
| fragrance | 0.100 |
| Polyoxyethylene 40 Stearate | 1.000 |
| Ultramarine Blue | 0.200 |
| mica and titanium dioxide (Mica - 23% Titanium Dioxide - 77%) | 0.400 |
| Sodium Sulfite | 0.100 |
| EDTA | 0.050 |
| l-naphthol | 0.375 |
| N,N bis (2-hydroxyethyl) PPD sulfate | 0.480 |
| resorcinol | 0.280 |
| p-phenylenediamine | 0.240 |
| deionized water | 67.775 |
| total | 100.000 |

0223572

## Example 2 - Light Blonde
### (Violet class shade)

|                                      | WT %    |
|--------------------------------------|---------|
| ammonium hydroxide                   | 9.000   |
| oleic acid                           | 10.500  |
| Lauramide DEA                        | 3.000   |
| cetyl alcohol                        | 2.500   |
| fragrance                            | 0.100   |
| Polysorbate-20                       | 0.500   |
| mica and titanium dioxide (mica - 23% $TiO_2$ - - 77% | 0.400   |
| Ultramarine Violet                   | 0.400   |
| sodium sulfite                       | 0.100   |
| EDTA                                 | 0.050   |
| m-aminophenol                        | 0.025   |
| resorcinol                           | 0.080   |
| p-phenylenediamine                   | 0.070   |
| p-aminophenol                        | 0.130   |
| deionized water                      | 73.145  |
| total                                | 100.000 |

### Example 3 - Light Violet Flame
(Red-Violet class shade)

| | WT % |
|---|---|
| ammonium hydroxide | 9.000 |
| cetyl alcohol | 10.000 |
| oleic acid | 2.000 |
| Synthetic Wax | 2.000 |
| Cocamide DEA | 3.000 |
| oleic acid | 3.000 |
| fragrance | 0.100 |
| <u>titanium dioxide coated mica with iron oxide</u> | 0.400 |
| (Iron Oxide - 40-47% | |
| $TiO_2$ - 2-4% | |
| 50-57% - mica) | |
| <u>mica and titanium dioxide and carmine</u> | 0.100 |
| (mica - 48-54% | |
| $TiO_2$ - 44-49% | |
| Carmine - 1.5-3.0%) | |
| sodium sulfite | 0.100 |
| EDTA | 0.050 |
| p-phenylenediamine | 0.245 |
| p-aminophenol | 0.330 |
| 4-amino-2-hydroxytoluene | 0.575 |
| deionized water | 69.100 |
| total | 100.000 |

### Example 4 - Light Auburn (Red-Orange class shade)

| | WT % |
|---|---|
| ammonium hydroxide | 8.000 |
| behenyl alcohol | 14.000 |
| behnic acid | 4.000 |
| Lauryl amine Oxide | 3.000 |
| Polyoxyethylene 50 lanolin derivative | 1.000 |
| beeswax | 1.000 |
| mieral oil | 2.000 |
| fragrance | 0.100 |
| mica and titanium dioxide (mica - 60-57% TiO$_2$ - 40-43%) | 0.200 |
| mica, titanium dioxide, Fe$_2$O$_3$ Fe$_2$O$_3$ - 5-7% TiO$_2$ - 35-40%  Balance - mica) | 0.100 |
| mica, titanium dioxide and iron oxide mica - 50-57% TiO$_2$ - 2-4% Iron Oxide - 40-47%) | 0.200 |
| Pigment Orange 16 | 0.100 |
| sodium sulfite | 0.100 |
| EDTA | 0.050 |
| resorcinol | 0.115 |
| p-phenylenediamine | 0.105 |
| p-aminophenol | 0.375 |
| 4-amino-2-hydroxytoluene | 0.360 |
| deionized water | 65.195 |
| total | 100.000 |

## Example 5 - Light Golden Blonde
### (gold class shade)

|                                          | WT %    |
|------------------------------------------|---------|
| ammonium hydroxide                       | 9.000   |
| oleic acid                               | 10.500  |
| Cocamide DEA                             | 3.000   |
| cetyl alcohol                            | 2.500   |
| fragrance                                | 0.100   |
| mica, titanium dioxide, $Fe_2O_3$        | 0.100   |
| $Fe_2O_3$ - 5-7%                         |         |
| $TiO_2$ - 35-40%                         |         |
| Balance - mica)                          |         |
| mica, titanium dioxide                   | 0.400   |
| mica - 60-57%                            |         |
| $TiO_2$ - 40-43%)                        |         |
| sodium sulfite                           | 0.100   |
| EDTA                                     | 0.050   |
| 1-naphthol                               | 0.015   |
| resorcinol                               | 0.060   |
| p-aminophenol                            | 0.300   |
| 2-methylresorcinol                       | 0.180   |
| deionized water                          | 73.695  |
|                                          |         |
| total                                    | 100.000 |

In each of the foregoing Examples 1-5 the oxidizing agent is 6.15% hydrogen peroxide solution. Hair is dyed by each of the dye compositions of Examples 1-5, by mixing 2 oz. of the composition of either of the Examples with 2 oz. of the oxidizing agent. The mixture is applied to the root area of hair for 20-40 min. The applied composition is combed or lathered through the hair shaft and ends for an additional 5-15 min. The hair is then thoroughly rinsed and then shampooed.

CLAIMS:

1.    An oxidation dye composition for dyeing hair, said composition containing oxidation dye intermediates which react in the presence of an oxidizing agent to develop color on hair, said composition containing a pigment component which is water-insoluble or substantially water-insoluble and which will be readily washed out of hair to which it is applied.

2.    A composition according to claim 1 wherein said pigment component serves as a color or color category identifying means for said composition.

3.    A composition according to claim 1/in which said composition, in the absence of said pigment component would be essentially colorless or would have a weak incidental color.
                                                   or Claim 2

4.    A composition according to/claims 1/wherein said pigment component comprises one or more pigments selected from the group consisting of inorganic pigments, organic pigments and mixtures thereof.
                              any one of      to 3

5.    A composition according to claim 4 wherein at least one of said pigments is a pearlized pigment.

6.    A composition according to claim 4/wherein the pigment component serves to identify a blue-gray color category.
                                           or Claim 5

7.    A composition according to claim 4/wherein the pigment component serves to identify a violet color category.
                                           or Claim 5

8.    A composition according to claim 4/wherein
the pigment component serves to identify a red-violet
color category.

*or Claim 5*

9.    A composition according to claim 4/wherein
the pigment component serves to identify a red-orange
color category.

*or Claim 5*

10.    A composition according to claim 4 wherein
the pigment component serves to identify a gold color
category.

11.    A composition according to any one of claims
1-10 in the form of a cream-based product or a gel.

12.    A composition according to any one of claims
1-10 in which said pigment component comprises    from
about 0.01% to about 10.0% by weight based on the
total weight of the composition.

13.    A composition according to any one of claims
1-10 in which said composition is in the form of a
cream-based product or gel and the pigment component
comprises from about 0.01% to about 10.0% by weight
based on the total weight of the composition.

14.    A hair coloring system comprising a plurality
of intermediate oxidation dye compositions for dyeing
hair, each of said compositions being contained in its
own container, each of said compositions containing
oxidation dye intermediates which react in the presence
of an oxidizing agent to develop color on hair, each of
said compositions containing a pigment component which
is water-insoluble or substantially water-insoluble and
which pigment component will be readily washed out of
hair to which it is applied, the pigment component in

- 18 -    0223572

each of said compositions being adapted to identify the
color or the color category for each of the hair
coloring dyes of said compositions in the system, after
their respective colors will have been developed on the
hair.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 086 442  (CHEMISCHE FABRIEK INDOLA COSMETICS) * Whole document * | 1-4,6-13 | A 61 K    7/13 |
| X | US-A-4 381 920  (GARLEN) * Whole document * | 1,4,11-13 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 11-02-1987 | FISCHER J.P. |